(19) Europäisches Patentamt
European Patent Office
Office européen des brevets

(11) Publication number: 0 166 221 B1

(12) # EUROPEAN PATENT SPECIFICATION

(45) Date of publication of patent specification: 23.01.91

(51) Int. Cl.$^5$: A 61 K 7/42, A 61 K 7/44, C 07 D 311/72

(21) Application number: 85106401.4

(22) Date of filing: 24.05.85

(54) **Sunscreen composition and method of use.**

(30) Priority: 29.05.84 US 614587
02.05.85 US 728005

(43) Date of publication of application:
02.01.86 Bulletin 86/01

(45) Publication of the grant of the patent:
23.01.91 Bulletin 91/04

(84) Designated Contracting States:
AT BE CH DE FR GB IT LI NL

(56) References cited:
DE-C- 724 268
US-A-4 454 112

CHEMICAL ABSTRACTS, vol. 84, 1976, Columbus, OH (US); p. 347, no. 155527k
"The Vitamins", 2nd edition, vol. V, 1972, page 193, W.H. SEBRELL, R.S. HARRIS, editors, Academic Press, New York, US; Chapter 16: "Tocopherols"

The file contains technical information submitted after the application was filed and not included in this specification

(73) Proprietor: HENKEL CORPORATION (a Delaware corp.)
7900 West 78th Street
Minneapolis Minnesota 55435 (US)

(72) Inventor: Tuominen, Francis W.
4656 Oregon Avenue North
Minneapolis, MN 55428 (US)

(74) Representative: von Kreisler, Alek, Dipl.-Chem. et al
Deichmannhaus am Hauptbahnhof
D-5000 Köln 1 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 166 221 B1

Courier Press, Leamington Spa, England.

## Description

This invention relates to the preparation of the ester of a tocopherol and certain acids and the use thereof in sunscreen compositions. The use of this ester provides a method of reducing the amount of ultraviolet radiation reaching the skin which method includes contacting the skin with an effective amount of the ester as a sunscreen agent. The ester may be applied in a composition containing other skin treating compositions including perfumes, thickeners, thinners, preservatives, antioxidants, surface active agents, solvents, water, moisturizers, stabilizers, counterirritants, anesthetizers, antiseptics, and the like.

## BACKGROUND OF THE INVENTION

Various tocopherol derivatives are known to exist. For instance United States Patent 2,231,125 issued to Karrer on February 11, 1941 describes the preparation of stearic and oleic esters of tocopherol. Karrer discusses the introduction of allyl groups into tocopherol in the United States Patent 2,245,480 issued June 10, 1941.

The preparation of tocopherol succinates by Baxter et al is found in United States Patent 2,358,046 issued September 12, 1944. The preparation of crystalline dl-alpha-tocopherol-p-nitro-benzoic acid esters is described in United States Patent 2,393,134 issued to Aeschlimann January 15, 1946. The process of esterifying tocopherols with acyl halides is set out in United States Patent 2,486,541 issued to Baxter et al on November 1, 1949.

Water soluble tocopherol derivatives prepared by esterifying tocopherol acid esters with polyethylene glycol are described in United States Patent 2,680,749 to Cawley et al on June 8, 1954. Among the tocopherol acid esters described by Cawley as suitable for condensation with the polyethylene glycol include: succinates, citraconates, methyl citraconates, itaconates maleates, glutaconates, and phthalates.

The preparation of tocopherol ascorbates is described by Spanel in United States Patent 3,151,127 issued September 29, 1964. United States Patent 3,869,477 issued March 4, 1975 to Schindo et al describes the preparation of tocopherol-p-chlorophenoxyisobutyric acid esters which are stated to have an affect on arteriosclerosis and of affecting betterment of lipid metabolism. Vitamin A acid esters of tocopherol are described in United States Patent 3,878,202 issued April 15, 1975 to Fukawa et al. Cholesterol esters of acetylsalicylate were stated to have been prepared by Montignie in the Bull. Soc. Chim. 49, 1852—1853 (1931).

U.S. Patent 4,154,823 discloses the use of p-aminobenzoic acid employed in a composition along with a tocopherol compound though not in the ester form. U.S. Patent 4,144,325 disclosed the use of esters such as glycerol-p-aminobenzoate ester.

The acetylsalicylate ester of tocopherol is described in my U.S. Patent 4,454,112.

## SUMMARY OF THE INVENTION

The present invention is concerned with certain acid esters of tocopherol and their use in a sunscreen composition in which the ester acts to absorb ultraviolet light. The benzoic acid esters, particularly tocopherol p-aminobenzoate, are particularly useful providing a method of reducing the amount of ultraviolet from reaching the skin when employed in an amount effective to do so, generally on the order of about 1 to 20% by weight of the composition in which it is employed, which composition includes an inert carrier and may also contain other skin conditioning or treating agents.

## DETAILED DESCRIPTION OF THE INVENTION

The present invention as previously stated relates to certain acid esters of tocopherol. The preferred form of tocopherol is that commonly denominated as alpha-tocopherol. The alpha-tocopherol may be either natural or synthetically produced. The natural Vitamin E occurs in the dextro form, d-α-tocopherol, which is the most preferred, while the synthetically produced contains a mixture of both forms "d" and levo, "l" forms. Other tocopherols may also be used in the present invention including the beta, gamma, and delta forms.

The acids which are employed to form the tocopherol esters are those which possess an unsaturated bond in conjugation with the carbonyl group. The preferred acids possessing this relationship are the aromatic acids such as benzoic acid, either substituted or unsubstituted. In such acids, the unsaturated bond in conjugation with the carbonyl group is a bond of the phenyl group itself. The acid may contain an alkenyl group between the carbonyl carbon and the phenyl group, with the unsaturation in the alkenyl group being in a conjugated relationship to the carbonyl group. Thus, aralkenyl groups of such type are satisfactory for the purposes of this invention. Cinnamic acid is illustrative thereof.

As indicated, the aryl or phenyl group may be substituted. A hydroxy substituent such as in hydroxy benzoic acid, i.e. salicyclic acid, or hydroxycinnamic acid is useful and said hydroxyl group may also be esterified with an acid, such as acetic acid to form the acetylsalicylic acid. Where the substituent is an amine group or substituted amine group, the acid is an aminobenzoic acid, the tocopherol ester of which is particularly useful in the present invention. Another illustration of a suitable acid is retinoic acid, sometimes referred to as Vitamin A acid as the structure corresponds to Vitamin A except that the alcohol form is now an acid form.

Ideally, the acids which are suitable herein for forming the tocopherol esters may be represented by the following formula:

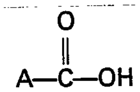

where A is an alkyl, cycloalkyl, aryl, alkaryl or aralkyl radical containing unsaturation which is in conjugation with the carbonyl group. A is desirably defined as:

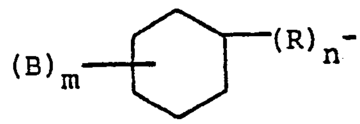

where R is an alkenyl group containing from 1—20 carbon atoms, preferably 1—12 carbon atoms; n is an integer of 0 or 1; B is a substituent which will not interfere with the esterification of the tocopherol, preferably an alkyl group, an amino group, hydroxyl or alkoxyl group, and m is an integer of 0, 1, 2 or 3. The alkyl group which may be represented by R′ preferably is a short chain group containing from 1—6 carbon atoms. The amino group is desirably represented as —N—$R_2''$ where R″ is H or R′ and when both are alkyl may be the same or different. The hydroxyl or alkoxyl substituted may ideally be represented as —OR″ where R″ is defined as above. The preferred B radicals are those selected from the groups consisting of —$NH_2$, —$NHCH_3$, —$N(CH_3)_2$, —OH, —$OCH_3$, —$OC_2H_5$. The preferred R alkenyl radicals are

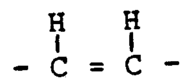

as in cinnamic acid or

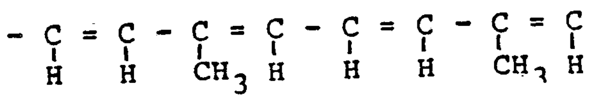

as in retinoic acid which also contains a cycloalkene group such as

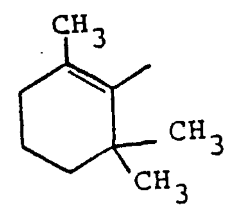

As indicated earlier, the tocopheryl p-aminobenzoate ester is particularly useful and the invention will be specifically exemplified by the preparation and formulation of this specific ester. It is understood however, that the other esters may be prepared by conventional esterification techniques, in which the acids will be employed in their halide or anhydride form.

The aminobenzoate portion of the ester is conveniently provided by reaction with the p-nitrobenzoyl halide in particular, p-nitrobenzoyl chloride, followed by reduction of the nitro group to an amino group.

The preparation is conveniently carried out by reacting equal molar quantities of the tocopherol and the p-nitrobenzoyl chloride using a solvent such as pyridine. While the reaction is conveniently carried out on an equal molar basis, it is also possible to include excess amounts of one ingredient or the other to ensure completion of the reaction. Thus, it is often desirable to include a slight excess of the chloride due to the fact that the tocopherol is the more expensive reactant. After completion of the reaction the solvent is removed and the product recovered, which can be purified by crystallization, i.e. from an alcohol such as isopropanol.

The product is then reduced in the conventional manner using a palladium catalyst and hydrogen under pressure. Palladium on carbon (5%) is a preferred catalyst, employing from 1—20% catalyst with about 10% based on weight of the nitrobenzoate employed. Pressures employed are generally above 50 psi. After completion of hydrogen uptake, the crude product is recovered with removal of the catalyst and solvent and the product purified by crystallization in a solvent such as acetone to provide the p-aminobenzoate ester of the tocopherol.

The general method of preparation of the ester is described in some detail in Swiss Patent 223,431 to F. Hoffmann-La Roche & Co., September 15, 1942, Chemical Abstracts, 1949, 1810.

As indicated, the esters find use as sunscreening agents to absorb a selected portion of the ultraviolet spectrum which is the major cause of sunburn. This portion of the spectrum, sometimes referred to as the UV-B portion as described in American Pharmacy, Vol. NS 21, No. 5, May 1981, pp. 294—298. In this utility of the present invention, namely that of ultraviolet absorption, the tocopherol p-aminobenzoate is found to

absorb ultraviolet radiation having a maximum absorbence at 298 nanometers. At one-half of the absorption maximum, the wave lengths at which the compound is absorbing ultraviolet radiation is from 284 to 314 nanometers. The compositions of the present invention are thus useful in absorbing a portion of the intense ultraviolet radiation at the specified wave lengths. That is, it must be remembered that the purpose of any sunscreening agent (UV absorber) is not to prevent the sun's ultraviolet radiation from reaching the skin, but to reduce its intensity so as to enable the skin to build up its own protection against exposure by means of tanning. Moreover, it has been suggested that ultraviolet radiation has the ability to induce skin carcinoma over a substantial portion of the ultraviolet spectrum. It is thus suggested that the use of the ester protects the skin from overdoses of ultraviolet radiation within the aforementioned wave length band.

It has also been noted with respect to the use of the ester as a sunscreen that there are purported effects of the use of tocopherols as skin conditioning agents. That is, the use of tocopherol and in particular alpha-tocopherol, that the skin retains a youthful appearance for a longer period of time than skin not so treated. This is particularly important when considering that the tocopherol esters of the present invention are suggested for use under adverse climactic conditions. That is, the compounds of the present invention when used as a sunscreen, not withstanding the effect of absorbing ultraviolet light, should provide skin care benefits due to the tocopherol portion of the molecule. Additionally, the tocopherol esters are not readily washed away from the skin and thus repeated applications of a sunscreen formulation containing the tocopherol esters are not frequently needed during sunbathing.

As indicated in the earlier background of the invention, p-aminobenzoic acid or the glycerol ester have been used in the past in composition for use as a sunscreening agent. With some persons these products have certain disadvantage and reactions which by means of the present invention are avoided by employing the tocopherol ester form. Thus, the present invention affords an advantage over the use of the acid alone.

The compositions of this invention are applied in any well known cosmetically and therapeutically acceptable carrier or vehicle, preferably of oily type, employed in sufficient amount to yield readily spreadable compositions with the consistency of an emulsion, lotion, oil, salve, ointment or the like. Illustrative of such carriers or vehicles are the liquid carriers such as mineral oils, silicone oils, liquid unsaturated alcohols, liquid wax esters, branched fatty alcohols and triglyceride oils, and mixtures thereof. Particularly preferred for this purpose are the mineral oils or normally liquid unsaturated vegetable oils, employed singly or in any desired mixtures thereof. Such oils, in which at least about 50% of the fatty acid components are mono- or polyunsaturated, are rapidly absorbed by the skin and thereby carry the essential vitamin components of the instant compositions into contact with burn-damaged cells or, when used for sunscreen purposes, prolong protective activity in that the absorbed or penetrated portions of the compositions resist removal from the skin surface by environmental and/or mechanical agencies. Generally about 25 to 50 parts of such oil or mixture thereof yields the desired consistency, those of lighter viscosity being preferred when a thinner, more rapidly penetrating composition is desired. As exemplary of such oils, there may be mentioned almond, apricot kernel, arachis, candlenut, castor, corn, cottonseed, croton, grapeseed, hazelnut, hempseed, olive, peach kernel, poppyseed, peanut, pumpkinseed, safflower, sesame, soybean, sunflower and walnut oils and the like.

The compositions of this invention may also contain small amounts of other known components of skin-treating compositions such as perfumes, thickeners, thinners, preservatives, antioxidants, surface active agents, coloring matter, organic solvents, water, stabilizers, demulcents, moisturizers, driers, stimulants, counterirritants anesthetizers, antiseptics, antibiotics, antipruritics, lubricants, coagulants, steroids, UV absorbers, and the like. Illustratively, from about 1 to 10 parts by weight of a relatively saturated, higher melting, more viscous vegetable oil such as coconut oil may be included for thickening and/or bulking effects. These oils, and the above-described unsaturated vegetable oils also often have UV absorbing properties. Smaller amounts may be included, for example, of lanolin as a penetrant, lubricant and anti-drying agent, lemon oil as a fragrance and neutralizer, camphor for stimulation, absorption, and removal of toxins from around the skin cells, Irish Moss as a thickener and demulcent, surfactants for solubilizing, emulsifying, dispersing, stabilizing and/or wetting functions, and water and organic solvents such as ethanol for solubilizing, thinning and the like.

Topical preparations will usually contain long chain alkanols in small amounts. The useful alkanols have from about 12—16 carbon atoms and may optionally contain one or more carbon to carbon double bonds and one or more branches in the molecule. Examples of alkanols are oleyl alcohol, cetyl alcohol 2-methyl undecanol, 2-hexyldecanol, 2-octyldodecanol, farnesol and phytol. Such preparations will also contain glycols or derivatives thereof such as hexylene glycol, diethylene glycol monostearates, glycerol. In lotion, emulsions and particularly microemulsions, sterols such as soya sterols and their alkoxylated forms such as ethoxylated phytosterols are employed.

The compositions of this invention are prepared in any desired manner and in any suitable order or sequence of addition of the various components and those skilled in the art will be readily cognizant of those available mixing procedures which are operative for the facile and expeditious production of such compositions. In use, the compositions are simply applied as a thin film or layer to the area of the skin which has been burned or which is to be protected from burning or reddening and left uncovered or covered as required or deemed advisable in any particular situation. These compositions may be

dispensed in or from any suitable container in bulk or pressurized in aerosol form. The preparations may be found in the form of solutions, oils, suspensions, gels, emulsions, ointments, pastes or sticks.

The following examples are only illustrative of preferred embodiments of my invention and are not to be regarded as limitative. All amounts and proportions referred to herein and in the appended claims are by weight unless otherwise indicated.

Example 1

Preparation of p-Nitrobenzoate of d-α-Tocopherol

The p-nitrobenzoate of d-α-tocopherol was prepared using the following materials:

| | Grams (g) |
|---|---|
| d-α-tocopherol (98% purity) | 50 |
| n-nitrobenzoyl chloride | 23 |
| pyridine | 200 |

The α-tocopherol was dissolved in the pyridine and the p-nitrobenzoyl chloride added in a single portion. The material turned blood red. The product was swirled at room temperature for 10 minutes and the color turned to a pale orange with the precipitation of pyridine hydrochloride. The reaction was warmed on a steam bath for 1 hour. There was then added 200 g of hexane followed by 500 g of water. The hexane phase was separated, washed with 5% hydrochloric acid solution to remove pyridine. The hexane phase was then washed with 10% potassium carbonate solution followed by water washing to neutrality. The solvent was removed in vacuo to give 64.4 g (96% yield) of crude p-nitrobenzoate of α-tocopherol.

14.5 g of the above material was crystallized from isopropyl alcohol to give a lemon yellow material with a melting point (mp) of 64—65°C.

Example 2

Preparation of p-Aminobenzoate of d-α-Tocopherol

The p-nitrobenzoate of d-α-tocopherol was prepared using the following materials:

| | |
|---|---|
| d-α-tocopherol p-nitrobenzoate (of Example 1) | 49.9 g |
| 5% Pd on carbon 50% w/w (Engelhard) | 5.0 g |
| Acetic Acid | 200 g |
| Hexane | 50 g |

The p-nitrobenzoate of d-α-tocopherol was dissolved in a mixture of hexane and acetic acid (50 g hexane: 200 g acid). This solution and 5.0 g of catalyst was placed in a Parr bottle and reduced with hydrogen at room temperature under 60 psi of $H_2$. The hydrogen uptake stopped after 20 min. The aminobenzoate had precipitated from solution. Acetone was added to dissolve the product. The catalyst was removed by filtration, then the mother liquor was diluted with water to precipitate the product. The resulting waxy material was suspended in isopropanol hexane (1:1) and washed with 5% potassium carbonate followed by water washing to neutrality. The solvent was removed in vacuo to give 35.0 g (74% yield). This crude material (yellowish solid) was crystallized from acetone to give a white solid (d-α-tocopherol p-aminobenzoate) mp 163—164°C.

Example 3

The product of Example 2 is formulated into a typical sunscreen lotion having the following composition:

| | |
|---|---|
| α-tocopherol p-aminobenzoate | 1% |
| oleyl alcohol | 10% |
| mineral oil | 89% |

The foregoing formulation is found effective in reducing the amount of ultraviolet radiation reaching the skin when applied at a level to about 0.25 grams per square centimeters of skin surface.

The following is a typical starting formulation for an oil in water sunscreen emulsion base:

| | % W/W |
|---|---|
| Mineral oil | 20.0 |
| Cetyl alcohol | 4.0 |
| Diethylene glycol monostearate | 3.0 |
| Ethoxylated soya sterols | 5.0 |
| Tocopherol ester | 1.5 |
| Water | to 100 |

To prepare, all the ingredients except for water are heated to about 80°C with agitation and the water at 80°C then added. After cooling, an alcohol such as ethanol is added, to provide desired consistency, as well as any preservatives and fragrances, to provide a soft liquid cream consistency, making it ideally suited for tube or squeeze bottle dispensing.

Another oil in water vehicle for topical application is a microemulsion lotion.

| | % |
|---|---|
| Ethoxylated soya sterols | 8.00 |
| Tocopherol ester | 5.00 |
| Mineral oil | 15.00 |
| Ethoxylated ether of oleyl alcohol | 10.00 |
| Hexylene glycol | 5.00 |
| Ethyl hexanediol | 2.00 |
| Water, deionized | to 100.00 |

**Claims for the Contracting States: BE CH DE FR GB IT LI NL**

1. A sunscreen composition comprising a carrier and an α-tocopherol ester of an acid possessing an unsaturated bond in conjugation with the carbonyl group, said ester being present in an effective amount to reduce the amount of ultraviolet radiation reaching the skin, characterized in that said α-tocopherol or a mixture thereof with beta, gamma and delta tocopherol is used, said α-tocopherol is d-α-tocopherol or a mixture of d and l-α-tocopherol, and said acid has the formula

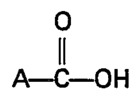

where A is an alkyl, cycloalkyl, aryl other than acetyl salicylic acid, alkaryl or aralkyl group containing the double bond in conjugation with the carbonyl group.

2. A composition as defined in claim 1 wherein A has the formula

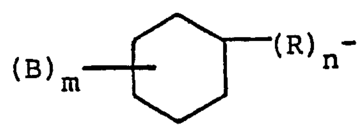

where R is an alkenyl group containing from 1—20 carbon atoms, n is an integer of 0 or 1, B is a substituent which will not interfere with esterification of tocopherol, and m is an integer of 0, 1, 2 or 3.

3. A composition as defined in claims 1 and 2 wherein B is selected from an alkyl group, amino group, hydroxyl group, alkoxyl group, $—NH_2$, $—NHCH_3$, $—N(CH_3)_2$, —OH, $—OCH_3$ and $—OC_2H_5$.

4. A composition as defined in claim 1 wherein said acid is a benzoic acid.

5. A composition as defined in claims 1 to 4 wherein said tocopherol ester is tocopherol p-aminobenzoate, preferably employed in an amount of about 1—20% by weight of said sunscreen composition.

6. A composition as defined in claims 1 to 5 wherein said carrier is employed in an amount of greater than 50% by weight of said sunscreen composition, and wherein said carrier is selected from mineral oil, silicone oil, liquid unsaturated alcohols, liquid wax esters, branched chain fatty alcohol, triglyceride oils and mixtures thereof.

7. A lotion containing the sunscreen composition as defined in claims 1 to 6.

8. A microemulsion containing the sunscreen composition of claims 1 to 6.

9. A cosmetic stick containing the sunscreen composition of claims 1 to 6.

**Claims for the Contracting State: AT**

1. Method for the preparation of a sunscreen composition comprising a carrier and an α-tocopherol ester of an acid possessing an unsaturated bond in conjugation with the carbonyl group, said ester being present in an effective amount to reduce the amount of ultraviolet radiation reaching the skin, wherein said α-tocopherol or a mixture thereof with beta, gamma and delta tocopherol is used, said α-tocopherol is d-α-tocopherol or a mixture of d and l-α-tocopherol, and said acid has the formula

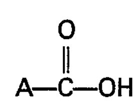

where A is an alkyl, cycloalkyl, aryl other than acetyl salicylic acid, alkaryl or aralkyl group containing the

double bond in conjugation with the carbonyl group by dissolving the α-tocopherol in pyridine, adding the acid and removal of the solvent after completion of the reaction.

2. Method according to claim 1 wherein A has the formula

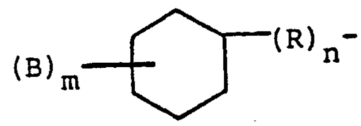

where R is an alkenyl group containing from 1—20 carbon atoms, n is an integer of 0 or 1, B is a substituent which will not interfere with esterification of tocopherol, and m is an integer of 0, 1, 2 or 3.

3. Method according to claims 1 and 2, wherein B is selected from an alkyl group, amino group, hydroxyl group, alkoxyl group, $—NH_2$, $—NHCH_3$, $—N(CH_3)_2$, $—OH$, $—OCH_3$ and $—OC_2H_5$.

4. Method according to claim 1 wherein said acid is a benzoic acid.

5. Method according to claims 1 to 4 wherein said tocopherol ester is tocopherol p-aminobenzoate, preferably employed in an amount of about 1—20% by weight of said sunscreen composition.

6. Method according to claims 1 to 5 wherein said carrier is employed in an amount of greater than 50% by weight of said sunscreen composition, and wherein said carrier is selected from mineral oil, silicone oil, liquid unsaturated alcohols, liquid wax esters, branched chain fatty alcohol, triglyceride oils and mixtures thereof.

7. Method for the preparation of a lotion containing the sunscreen composition prepared according to claims 1 to 6 by heating all the ingredients to about 80°C with agitation, then adding water and then, after cooling adding alcohol to provide the desired consistency.

8. Method for the preparation of a microemulsion containing the sunscreen composition prepared according to claims 1 to 6 in a conventional manner.

9. Method for the preparation of a cosmetic stick containing the sunscreen composition prepared according to claims 1 to 6 in a conventional manner.

**Patentansprüche für den Vertragsstaat: BE CH DE FR GB IT LI NL**

1. Sonnenschutz-Zusammensetzung umfassend einer Träger und einen α-Tocopherolester einer Säure, welche eine ungesättigte Bindung in Konjugation mit der Carbonylgruppe besitzt, wobei der Ester in einer zur Verminderung des Anteils der die Haut erreichenden ultravioletten Strahlung wirksamen Menge vorhanden ist, dadurch gekennzeichnet, daß das α-Tocopherol oder ein Gemisch desselben mit beta-, gamma- und delta-Tocopherol verwendet wird, daß das α-Tocopherol d-α-Tocopherol oder ein Gemisch aus d- und l-α-Tocopherol ist und daß die Säure die Formel

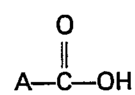

besitzt, worin A eine Alkyl-, Cycloalkyl-, eine andere Arylgruppe als Acetylsalicylsäure, eine Alkaryl- oder eine Aralkylgruppe ist, welche die Doppelbindung in Konjugation mit der Carbonylgruppe enthält.

2. Zusammensetzung wie in Anspruch 1 bezeichnet, wobei A die Formel

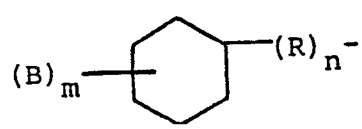

hat, worin R eine Alkenylgruppe ist, welche 1—20 Kohlenstoff-Atome enthält, n eine ganze Zahl 0 oder 1 ist, B ein Substituent ist, welcher die Veresterung von Tocopherol nicht beeinträchtigt, und m eine ganze Zahl 0, 1, 2 oder 3 ist.

3. Zusammensetzung wie in den Ansprüchen 1 und 2 bezeichnet, wobei B aus einer Alkylgruppe, Aminogruppe, Hydroxylgruppe, Alkoxylgruppe, $—NH_2$, $—NHCH_3$, $—N(CH_3)_2$, $—OH$, $—OCH_3$ und $—OC_2H_5$ ausgewählt ist.

4. Zusammensetzung wie in Anspruch 1 bezeichnet, worin die Säure eine Benzoesäure ist.

5. Zusammensetzung wie in den Ansprüchen 1 bis 4 bezeichnet, worin der Tocopherolester Tocopherol-p-aminobenzoat ist und vorzugsweise in einer Menge von etwa 1—20 Gew.-% der Sonnenschutz-Zusammensetzung angewandt wird.

6. Zusammensetzung wie in den Ansprüchen 1 bis 5 bezeichnet, worin der Träger in einer Menge von mehr als 50 Gew.-% der Sonnenschutz-Zusammensetzung angewandt wird und worin der Träger aus Mineralöl, Silikonöl, flüssigen ungesättigten Alkoholen, flüssigen Wachsestern, verzweigtkettigen Fettalkoholen, Triglyceridölen und deren Gemischen ausgewählt ist.

7. Lotion enthaltend die in den Ansprüchen 1 bis 6 bezeichnete Sonnenschutz-Zusammensetzung.

8. Mikroemulsion enthaltend die in den Ansprüchen 1 bis 6 bezeichnete Sonnenschutz-Zusammensetzung.

9. Kosmetischer Stift enthaltend die in den Ansprüchen 1 bis 6 bezeichnete Sonnenschutz-Zusammensetzung.

**Patentansprüche für den Vertragsstaat: AT**

1. Verfahren zur Herstellung einer Sonnenschutz-Zusammensetzung umfassend einer Träger und einen α-Tocopherolester einer Säure, welche eine ungesättigte Bindung in Konjugation mit der Carbonylgruppe besitzt, wobei der Ester in einer zur Verminderung des Anteils der die Haut erreichenden ultravioletten Strahlung wirksamen Menge vorhanden ist, wobei das α-Tocopherol oder ein Gemisch desselben mit beta-, gamma- und delta-Tocopherol verwendet wird, das α-Tocopherol d-α-Tocopherol oder ein Gemisch aus d- und l-α-Tocopherol ist und die Säure die Formel

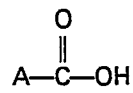

besitzt, worin A eine Alkyl-, Cycloalkyl-, eine andere Arylgruppe als Acetylsalicylsäure, eine Alkaryl- oder eine Aralkylgruppe ist, welche die Doppelbindung in Konjugation mit der Carbonylgruppe enthält, durch Lösen des α-Tocopherols in Pyridin, Zugabe der Säure und Entfernen des Lösungsmittels nach Vervollständigung der Reaktion.

2. Verfahren gemäß Anspruch 1, wobei A die Formel

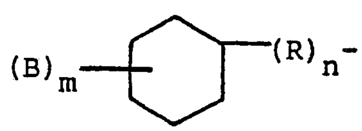

hat, worin R eine Alkenylgruppe ist, welche 1—20 Kohlenstoff-Atome enthält, n eine ganze Zahl 0 oder 1 ist, B ein Substituent ist, welcher die Veresterung von Tocopherol nicht beeinträchtigt, und m eine ganze Zahl 0, 1, 2 oder 3 ist.

3. Verfahren gemäß Anspruch 1 und 2, wobei B aus einer Alkylgruppe, Aminogruppe, Hydroxygruppe, Alkoxylgruppe, $—NH_2$, $—NHCH_3$, $—N(CH_3)_2$, $—OH$, $—OCH_3$ und $—OC_2H_5$ ausgewählt ist.

4. Verfahren gemäß Anspruch 1, worin die Säure eine Benzoesäure ist.

5. Verfahren gemäß den den Ansprüchen 1 bis 4, worin der Tocopherolester Tocopherol-p-aminobenzoat ist und vorzugsweise in einer Menge von etwa 1—20 Gew.-% Sonnenschutz-Zusammensetzung angewandt wird.

6. Verfahren gemäß den Ansprüchen 1 bis 5, worin der Träger in einer Menge von mehr als 50 Gew.-% der Sonnenschutz-Zusammensetzung angewandt wird und worin der Träger aus Mineralöl, Silikonöl, flüssigen ungesättigten Alkoholen, flüssigen Wachsestern, verzweigtkettigen Fettalkoholen, Triglyceridölen und deren Gemischen ausgewählt ist.

7. Verfahren zur Herstellung einer Lotion enthaltend die gemäß den Ansprüchen 1 bis 6 hergestellte Sonnenschutz-Zusammensetzung durch Erhitzen der gesamten Bestandteile unter Rühren auf etwa 80°C, dann Zugabe von Wasser und dann nach Kühlung Zugabe von Alkohol zur Erzielung der gewünschten Konsistenz.

8. Verfahren zur Herstellung einer Mikroemulsion enthaltend die gemäß den Ansprüchen 1 bis 6 hergestellte Sonnenschutz-Zusammensetzung auf übliche Weise.

9. Verfahren zur Herstellung eines kosmetischen Stifts enthaltend die gemäß den Ansprüchen 1 bis 6 hergestellte Sonnenschutz-Zusammensetzung auf übliche Weise.

**Revendications pour les Etats contractants: BE CH DE FR GB IT LI NL**

1. Une composition formant écran solaire, comprenant un véhicule et un ester d'α-tocophérol d'un acide possédant une liaison insaturée en conjugaison avec le groupe carbonyle, ledit ester étant présent en une quantité efficace pour réduire la quantité de rayonnement ultraviolet atteignant la peau, caractérisée en ce que l'on utilise ledit α-tocophérol ou un mélange de celui-ci avec le β-, γ- et δ-tocophérol, ledit α-tocophérol est le d-α-tocophérol ou un mélange de d- et de l-α-tocophérol, et ledit acide répond à la formule

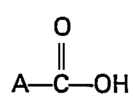

dans laquelle A est un groupe alcoyle, cycloalcoyle, aryle autre que l'acide acétylsalicylique, alcaryle ou aralcoyle contenant la double liaison en conjugaison avec le groupe carbonyle.

2. Une composition selon la revendication 1, dans laquelle A a pour formule

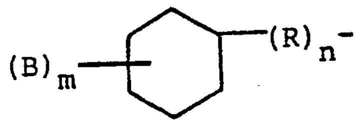

dans laquelle R est un groupe alcényle contenant 1 à 20 atomes de carbone, n est un entier valant 0 ou 1, B est un substituant qui ne gêne pas l'estérification du tocophérol et m est un entier valant 0, 1, 2 ou 3.

3. Une composition comme défini dans les revendications 1 et 2, dans laquelle B est choisi parmi un groupe alcoyle, un groupe amino, un groupe hydroxyle, un groupe alcoxyle, $—NH_2$, $—NHCH_3$, $—N(CH_3)_2$, —OH, $—OCH_3$ et $—OC_2H_5$.

4. Une composition comme défini dans la revendication 1, dans laquelle ledit acide est un acide benzoïque.

5. Une composition comme de défini dans les revendications 1 à 4, dans laquelle ledit ester de tocophérol est le p-aminobenzoate de tocophérol utilisé de préférence en une quantité d'environ 1 à 20% du poids de ladite composition formant écran solaire.

6. Une composition comme défini dans les revendications 1 à 5, dans laquelle ledit véhicule est utilisé en une quantité supérieure à 50% du poids de ladite composition formant écran solaire et dans laquelle ledit véhicule est choisi parmi une huile minérale, une huile de silicone, les alcools insaturés liquides, les esters de cire liquides, un alcool gras à chaîne ramifiée, les huiles de triglycéride et leurs mélanges.

7. Une lotion contenant la composition formant écran solaire comme défini dans les revendications 1 à 6.

8. Une microémulsion contenant la composition formant écran solaire des revendications 1 à 6.

9. Un bâton cosmétique contenant la composition formant écran solaire des revendications 1 à 6.

**Revendications pour Etat contractant: AT**

1. Procédé pour la préparation d'une composition formant écran solaire, comprenant un véhicule et un ester d'α-tocophérol d'un acide possédant une liaison insaturée en conjugaison avec le groupe carbonyle, ledit ester étant présent en une quantité efficace pour réduire la quantité de rayonnement ultraviolet atteignant la peau, dans lequel on utilise ledit α-tocophérol ou un mélange de celui-ci avec le β-, γ- et δ-tocophérol, ledit α-tocophérol est le d-α-tocophérol ou un mélange de d- et de l-α-tocophérol, et ledit acide formule

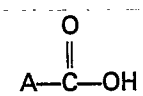

dans laquelle A est un groupe alcoyle, cycloalcoyle, aryle autre que l'acide acétylsalicylique, alcaryle ou aralcoyle contenant la double liaison en conjugaison avec le groupe carbonyle, par dissolution de l'α-tocophérol dans la pyridine, addition de l'acide et élimination du solvant après achèvement de la réaction.

2. Procédé selon la revendication 1, dans lequel A a pour formule

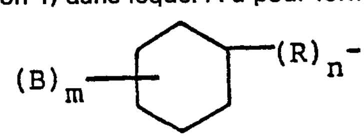

dans laquelle R est un groupe alcényle contenant 1 à 20 atomes de carbone, n est un entier valant 0 ou 1, B est un substituant qui ne gêne pas l'estérification du tocophérol et m est un entier valant 0, 1, 2 ou 3.

3. Procédé selon les revendications 1 et 2, dans lequel B est choisi parmi un groupe alcoyle, un groupe amino, un groupe hydroxyle, un groupe alcoxyle, $—NH_2$, $—NHCH_3$, $—N(CH_3)_2$, —OH, $—OCH_3$ et $—OC_2H_5$.

4. Procédé selon la revendication 1, dans lequel ledit acide est un acide benzoïque.

5. Procédé selon les revendications 1 à 4, dans lequel ledit ester de tocophérol est le p-aminobenzoate de tocophérol utilisé de préférence en une quantité d'environ 1 à 20% du poids de ladite composition formant écran solaire.

6. Procédé selon les revendications 1 à 5, dans lequel ledit véhicule est utilisé en une quantité supérieure à 50% du poinds de ladite composition formant écran solaire et dans lequel ledit véhicule est choisi parmi une huile minérale, une huile de silicone, les alcools insaturés liquides, les esters de cire liquides, un alcool gras à chaîne ramifiée, les huiles de triglycéride et leurs mélanges.

7. Procédé pour la préparation d'une lotion, contenant la composition formant écran solaire préparée selon les revendications 1 à 6, par chauffage de tous les ingrédients à environ 80°C avec agitation, puis addition d'eau, puis, après refroidissement, addition d'alcool pour établir la consistance désirée.

8. Procédé pour la préparation d'une microémulsion, contenant la composition formant écran solaire selon les revendications 1 à 6, de façon classique.

9. Procédé pour la préparation d'une bâton cosmétique, contenant la composition formant écran solaire selon les revendications 1 à 6, de façon classique.